# EUROPÄISCHE PATENTSCHRIFT

(11) **EP 2 621 464 B1**
(45) Veröffentlichungstag und Bekanntmachung des Hinweises auf die Patenterteilung: **07.03.2018**
(21) Anmeldenummer: 11760481.9
(22) Anmeldetag: 22.09.2011
(51) Int. Cl.: A61K 8/89, A61Q 5/12, A61Q 5/00, A61K 8/891

(54) **PFLEGEPRODUKTE ZUM SCHÜTZEN COLORIERTER HAARE MIT SILIKONHARZEN**
CARE PRODUCTS FOR PROTECTING DYED HAIR, COMPRISING SILICONE RESINS
PRODUITS DE SOIN POUR PROTÉGER DES CHEVEUX COLORÉS COMPRENANT DES RÉSINES DE SILICONE

(30) Priorität: 01.10.2010 DE 102010041887
(43) Veröffentlichungstag der Anmeldung: 07.08.2013
(73) Patentinhaber: Beiersdorf AG, 20253 Hamburg (DE)
(72) Erfinder: WÄCHTER, Anna Christina, 20251 Hamburg (DE); KLAUCK, Robert, 21465 Reinbek (DE)
(74) Vertreter: Hartmann, Jost
(86) Internationale Anmeldenummer: PCT/EP2011/066476
(87) Internationale Veröffentlichungsnummer: WO 2012/041756

(56) Entgegenhaltungen:
- EP-A1- 2 263 647
- WO-A1-99/55295
- WO-A2-2010/014352
- US-A- 4 983 383
- US-A- 5 733 537
- US-A1- 2006 280 693
- US-A1- 2007 166 271
- US-A1- 2007 269 389
- US-A1- 2009 074 702
- US-A1- 2009 214 458
- US-A1- 2009 285 765
- US-A1- 2010 015 078
- Dow Corning: "Light Touch Sports Sunscreen", , 22. August 2010 (2010-08-22), XP002670916, Gefunden im Internet: URL:http://www.dowcorning.com/content/publ ishedlit/FORMUL_01448.pdf [gefunden am 2012-03-07]
- Dow Corning: "Comfort Stay Foundation", , 6. August 2010 (2010-08-06), XP002670917, Gefunden im Internet: URL:http://www.dowcorning.com/content/publ ishedlit/FORMUL_01447.pdf [gefunden am 2012-02-07]

## Beschreibung

Manche Menschen färben sich die Haare. Gefällt das Ergebnis, so entsteht der Wunsch, die Farbe möge lange so bleiben, wie sie ist. Nach dem Färben der Haare verblasst die Haarfarbe durch Umwelteinflüsse wie z. B. Sonneneinstrahlung, mechanische und thermische Beanspruchungen wie Kämmen und Föhnen, aber auch durch den Haarwaschvorgang, welcher den größten negativen Effekt auf die Brillanz und Intensität der Haarfarbe darstellt. Ziel eines Pflegeproduktes für gefärbtes Haar ist es, die Haare optimal vor diesen Einflüssen zu schützen.

Standard Pflegeprodukte für coloriertes Haar enthalten oft UV-Filter, um das Verblassen der Haare durch Sonneneinstrahlung zu schützen.

Vor dem eigentlich gravierenderen Einfluss, dem Haarwaschvorgang, werden die Haare nicht ausreichend geschützt.

Haarfarben im Sinne der vorliegenden Schrift sind semipermanente Haarfarben (wie z. B. Schwarzkopf Brilliance Tönungs-Creme, L'Oréal Si Naturelle Creme Schaum Tönung, Alcina Color Emulsion, Goldwell Elumen) oder permanente Haarfarben (wie z. B. Garnier Herba Shine, Garnier Nutrisse Creme, Garnier Color Intense, L'Oréal Recital Préférence, L'Oréal Excell 10, L'Oréal Excellence Creme, Schwarzkopf Essential Color, Schwarzkopf Diadem, Schwarzkopf Brilliance Intensiv Color Creme, Schwarzkopf Country Colors, Schwarzkopf Live Color XXL).

Silikonharze im Sinne der vorliegenden Schrift sind vernetzte Polysiloxane. Man unterscheidet Silikonharze anhand verschiedener Monomereinheiten wie folgt: R₃SiO_{1/2} (M), R₂SiO_{2/2} (D), RSiO_{3/2} (T), SiO_{4/2} (Q).

Von besonderer Bedeutung sind Trimethylsiloxysilicate (MQ-Silikonharz) bzw. Mischungen aus Trimethylsiloxysilicate und Polypropylsilsesquioxane (MQ/TPr-Silikonharz).

Silikonharze werden weit verbreitet in der Automobilpfilege, in Haushalts- und Lederpflegemitteln eingesetzt; aber auch im Kosmetikbereich finden sie bereits Anwendung.

Sie werden besonders für Produkte eingesetzt, in denen eine gute Wasserresistenz und filmformende Eigenschaften im Fokus stehen, was z. B. bei Babycremes, schützenden Cremes und bestimmten Make-up Produkten der Fall ist.

Die Schrift EP 2 016 933 A1 offenbart bereits eine wasserfreie Haarbehandlungszubereitung mit einem Copolymer aus Silikonharzen und flüssigen Silikonen, sowie weiteren Silikonen wie flüchtigen Silikonen. Die Schrift EP 0 560 879 B1 offenbart bereits eine Haarbehandlungszubereitung mit 0,1 % bis 10 % dispergiertem Silikon, dass unter anderem bestimmte silikonlösliche, aber wasserunlösliche Silikonharze enthält. Die Schrift EP 1 119 339 B1 offenbart bereits Shampoos mit Siloxysilikaten. Die Schrift US 20070269389 A1 offenbart bereits Zubereitungen mit Filmbildnersystemen, die auch Trimethylsiloxysilikate enthalten können. Die Schrift WO 2009/080964 A3 offenbart bereits Schminkprodukte mit bestimmten Silikonharzen. Die Schrift US 7601179 B2 offenbart bereits ein Verfahren zum Schutz keratinischer Fasern, dass Silsesquioxane oder Trimethylsiloxysilikate in Kombination mit einem Nylon 611/Dimethicone Copolymer zum Einsatz kommen lässt. Die Schriften WO 2005/075542 A1 und WO 2005/075567 A1 offenbaren bereits spezielle Silikonharze. Die Schrift WO 2005/100444 A1 offenbart bereits Silsesquioxane Harzwachse. US 2009/0074702 A1 offenbart Methoden, um die Farbe von künstlich gefärbtem Haar zu erhalten. Dazu werden Zubereitungen auf das Haar aufgetragen, die film-bildende Silikonharze und ein Polyamid-Silikone-Copolymer enthalten. Es bildet sich ein Film auf dem Haar, der die künstliche Farbe auf dem Haar fixieren soll. WO 99/55295 A1 beschreibt auch eine Methode, um bei gefärbtem Haar ein Verblassen oder eine Farbveränderung der künstlichen Farbe zu verhindern. Dazu wird das Haar mit einer Zubereitung behandelt, die ein hydrophobes und/oder kationisches Mittel enthält. Das hydrophobe Mittel ist eine lösliche oder unlösliche Silikonverbindung.

All dies konnte die Mängel des Standes der Technik nicht beseitigen. Es fehlte dem Stand der Technik an Zubereitungen, die die Haarfarbe vor dem Auswaschen schützen, ohne dabei sensorisch nicht akzeptable Nebeneffekte wie etwa fettende Haare hervorzurufen. Überraschend und für den Fachmann nicht vorhersehbar hat sich nun herausgestellt, dass die Verwendung von Haarbehandlungsmitteln enthaltend
i) 0 bis 1 Gew. % anionische Tenside,
ii) 0,5 bis 5 Gew.-% Silikonharze
   wobei die Silikonharze
   a) M- und Q-Monomereinheiten im Verhältnis M:Q = 0,4 : 1 bis 1,2 : 1 enthalten,
   b) eine Molmasse von 12 bis 35 kg/mol, besonders bevorzugt 15 bis 32 kg/mol haben
   c) und zusätzlich T- oder D-Monomereinheiten enthalten,
   wobei die Silikonharze in Form von Trimethylsiloxysilicaten und
   Polypropylsilsesquioxane vorliegen,
   wobei die Gesamtmenge an Silikonharz im Bereich von 0,5 bis 5 Gew. % liegt,
iii) Polydimethylsiloxane,
iv) 0 bis 0,5 Gew. % Wasser,
zum Schutz der Farbe künstlich gefärbter Haare vor dem Verblassen der Farbe, den Mängeln des Standes der Technik abhilft.

Durch die Verwendung von Haarpflegeformulierungen gemäß Anspruch 1 ist es aus verschiedenen Applikationsformen möglich (rinse-off, leave-on), einen okklusiven Film auf dem Haar zu hinterlassen, der die Haarfarbe vor dem Auswaschen mit Wasser und Shampoo schützt. Haare, die mit semipermanenten Haarfarben behandelt sind, lassen sich durch die erfindungsgemäßen Zubereitungen besonders gut schützen.

Das Silikonharz enthält M- und Q-Monomereinheiten im Verhältnis
M : Q = 0,4 : 1 bis 1,2 : 1. Solche Verhältnisse von Monomereinheiten zueinander sind als molare Verhältnisse zu verstehen. Das entsprechende Gewichtsverhältnis ist
M : Q = 0.5 ; 1.0 bis 1.6 : 1.0. Bevorzugt ist es, wenn das Gewichtsverhältnis M : Q = 0.9 bis 1.4 ist.

Das Silikonharz hat eine Molmasse von 12 bis 35 kg/mol, bevorzugt 15 bis 32 kg/mol.

Das Silikonharz enthält zusätzlich T- oder D-Monomereinheiten. Bevorzugt ist es, wenn das Silikonharz M- und Q- und entweder T- oder D-Monomereinheiten enthält deren Verhältnis M:Q sich wie 0,5 : 1 bis 1 : 1 verhält und der Gewichtsanteil *an D*- oderT-Einheiten 30 bis 70 Gew.-% beträgt.

Als T- oder D-Monomereinheiten werden T-Propyl- oder D verwendet.

Also ist neben Trimethylsiloxysilicate Polypropylsilsesquioxane enthalten.

Besonders bevorzugt sind als Silikonharze die Produkte Trimethylsiloxysilicate + Cyclomethicone, Momentive SS 4230, Trimethylsiloxysilicate + Dimethicone, Dow Corning 593 Fluid, blend aus Trimethylsiloxysilicate und Polypropylsilsesquioxane, Dow Corning MQ-1640.

Es ist zusätzlich Polydimethylsiloxan enthalten, bevorzugt ein lineares Polydimethylsiloxan mit geringer Viskosität (90 bis 110 mm²/s) und geringem Molekulargewicht enthalten ist, besonders bevorzugt in Konzentrationen von 2 bis 7 Gew.-%, ganz besonders bevorzugt in einer Konzentration von 4,6 Gew.-%, und die Zubereitung dabei 0 bis 0,5 Gew.-% Wasser enthält. Besonders bevorzugt ist es, wenn zusätzlich 0,2 bis 2 Gew.-% Polypropylsilsesquioxane enthalten ist.

Solche Zubereitungen können besonders vorteilhaft als so genannte Seren formuliert werden. Die Erfindung umfasst auch die Verwendung von erfindungsgemäßen Zubereitungen zur Verringerung der eletrostatischen Aufladung von Haaren und zur Verbesserung der Nass- und Trockenkämmbarkeit, der Nass- und Trockenentwirrbarkeit, der Nass und Trockengleitfähigkeit. Weiter umfasst die Erfindung ein Verfahren zum Schutz der Farbe künstlich gefärbter Haare vor dem Verblassen der Farbe dadurch, dass die Haare durch Auftragen einer erfindungsgemäßen Zubereitung mit Silikonharz beschichtet werden, wobei die Zubereitung nicht ausgespült wird.

### Beispiele mit MQ-Silikonharzen

| INCI | Gew.-% (bezogen auf den Aktivgehalt) | | | | | | |
|---|---|---|---|---|---|---|---|
| | 1* (rinse-off Condition er 1) | 2* (rinse-off Condition er 2) | 3* (rinse-off condition er 3) | 4* (leave -on 1) | 5* (leave -on 2) | 6** (leave -on 3) | 7** (leave -on 4) |
| 1.Coco Betaine | 0,26 | 0,26 | 0,26 | 0 | 0 | 0 | 0 |
| 2, Cetearyl Alkohol | 0 | 0 | 0 | 0 | 0 | 0 | 0 |
| 3. Citric Acid | 0 | 0 | 0 | 0 | 0 | 0 | 0 |
| 4. Distearoylethyl Hydroxyethylmonium Methosulfate + Cetearyl Alkohol | 0 | 0 | 0 | 0 | 0 | 0 | 0 |
| 5. *Palmitamidopropyltrimoni* um Chloride + Propylene Glykol | 0 | 0 | 0 | 0 | 0 | 0 | 0 |
| 6. Hydroxypropyl Methylcellulose | 0 | 0 | 0 | 0 | 0 | 0 | 0 |
| 7. Methylparaben | 0,25 | 0,25 | 0,25 | 0 | 0 | 0 | 0 |
| DMDM Hydantoin | 0 | 0 | 0 | 0,6 | 0,6 | 0 | 0 |
| 9. Propylparaben | 0,1 | 0,1 | 0,1 | 0 | 0 | 0 | 0 |
| 10. Sodium Benzoate | 0 | 0 | 0 | 0 | 0 | 0 | 0 |
| 11. Lactic Acid | 0,72 | 0,72 | 0,72 | 0 | 0 | 0 | 0 |
| 12. Sodium Chloride | 0,01 | 0,01 | 0,01 | 0 | 0 | 0 | 0 |
| 13. Polyquaternium-37 | 0 | 0 | 0 | 0 | 0 | 0 | 0 |
| 14. Cetrimonium Chloride | 0 | 0 | 0 | 0 | 0 | 0 | 0 |
| 15. Cetyl Alkohol | 2,2 | 2,2 | 2,2 | 0 | 0 | 0 | 0 |
| 16. Stearyl Alkohol | 4,4 | 4,4 | 4,4 | 0 | 0 | 0 | 0 |
| 17. Dimethicone | 5,4 | 10 | 3 | 0 | 4,6 | 4,6 | 0 |
| 18. Stearamidopropyl Dimethylamine | 2,2 | 2,2 | 2,2 | 0 | 0 | 0 | 0 |
| 19. Trimethylsiloxysilicate | 2,5 | 5 | 0 | 2,5 | 2,5 | 2,5 | 0 |
| 20. Cyclomethicone | 0 | 0 | 0 | 3,1 | 0 | 87,6 | 94,8 |
| 21. Dimethiconol | 0 | 0 | 0 | 0 | 0 | 5,2 | 5,2 |
| 22. Glyceryl Isostearate | 0 | 0 | 0 | 2,2 | 2,2 | 0 | 0 |
| 23. Isoceteth-20 | 0 | 0 | 0 | 5 | 5 | 0 | 0 |
| 24. Glycerin | 0 | 0 | 0 | 2,6 | 2,6 | 0 | 0 |
| 25. Cocamidopropyl Betaine | 0 | 0 | 0 | 0 | 0 | 0 | 0 |
| 26. Sodium Laureth Sulfate | 0 | 0 | 0 | 0 | 0 | 0 | 0 |
| 27. Polyquaternium-10 | 0 | 0 | 0 | 0 | 0 | 0 | 0 |
| 28. C12-15 Alkyl Benzoate | 0 | 0 | 0 | 0 | 0 | 0 | 0 |
| 29. Alcohol Denat. | 0 | 0 | 0 | 0 | 0 | 0 | 0 |
| 30. Cl 42090 | 0 | 0 | 0 | 0 | 0 | 0 | 0 |
| antioxidant, pHadjustments, parfum | Qs | qs | qs | qs | qs | qs | qs |
| Water | Ad 100 | Ad 100 | Ad 100 | Ad 100 | Ad 100 | 0 | 0 |

| | | | | | | | |
|---|---|---|---|---|---|---|---|
| * nicht erfindungsgemäßes Beispiel ** Vergleichsbeispiel | | | | | | | |

| INCI | Gew.-% (bezogen auf den Aktivgehalt) | | | | | |
|---|---|---|---|---|---|---|
| | 8* (rinse-off conditioner 4) | 9* (leave-on 5) | 10* (leave-on 6) | 11* (leave-on 7) | 12* (Shampoo) | 13* (leave-on 8) |
| 1.Coco Betaine | 0,26 | 0 | 0 | 0 | 0 | 0 |
| 2, Cetearyl Alkohol | 0 | 0 | 0 | 2,8 | 0 | 0 |
| 3. Citric Acid | 0 | 0 | 0 | 0,01 | 0 | 0,05 |
| 4. Distearoylethyl Hydroxyethylmonium Methosulfate + Cetearyl Alkohol | 0 | 0 | 0 | 2 | 0 | 0 |
| 5. Palmitamidopropyltrimonium Chloride + Propylene Glycol | 0 | 0 | 0 | 1 | 0 | 0 |
| 6. Hydroxypropyl Methylcellulose | 0 | 0 | 0 | 0,25 | 0 | 0 |
| 7. Methylparaben | 0,25 | 0 | 0 | 0,25 | 0 | 0 |
| 8. DMDM Hydantoin | 0 | 0,6 | 0 | 0 | 0 | 0 |
| 9. Propylparaben | 0,1 | 0 | 0 | 0,1 | 0 | 0 |
| 10. Sodium Benzoate | 0 | 0 | 0 | 0 | 0,4 | 0,4 |
| 11. Lactic Acid | 0,72 | 0 | 0 | 0 | 0 | 0 |
| 12. Sodium Chloride | 0,01 | 0 | 0 | 0 | 0 | 0 |
| 13. Polyquaternium-37 | 0 | 2 | 0 | 0 | 0 | 0 |
| 14. Cetrimonium Chloride | 0 | 0,6 | 0 | 0 | 0 | 0 |
| 15. Cetyl Alkohol | 2,2 | 0 | 0 | 0 | 0 | 0 |
| 16. Stearyl Alkohol | 4,4 | 0 | 0 | 0 | 0 | 0 |
| 17. Dimethicone | 5,4 | 4,6 | 4,6 | 5 | 5,4 | 4,6 |
| 18. Stearamidopropyl Dimethylamine | 2,2 | 0 | 0 | 0 | 0 | 0 |
| 19. Trimethylsiloxysilicate | 1 | 2,5 | 1 | 2,5 | 2,5 | 2,5 |
| 20. Cyclomethicone | 0 | 0 | 89,2 | 0,5 | 0 | 25,4 |
| 21. Dimethiconol | 0 | 0 | 5,2 | 0 | 0 | 0 |
| 22. Glyceryl Isostearate | 0 | 0 | 0 | 0 | 0 | 0 |
| 23. Isoceteth-20 | 0 | 0 | 0 | 0 | 0 | 0 |
| 24. Glycerin | 0 | 0 | 0 | 8,7 | 0 | 0 |
| 25. Cocamidopropyl Betaine | 0 | 0 | 0 | 0 | 3 | 0 |
| 26. Sodium Laureth Sulfate | 0 | 0 | 0 | 0 | 9 | 0 |
| 27. Polyquaternium-10 | 0 | 0 | 0 | 0 | 0,3 | 0 |
| 28. C12-15 Alkyl Benzoate | 0 | 0 | 0 | 0 | 0 | 3,5 |
| 29. Alcohol Denat. | 0 | 0 | 0 | 0 | 0 | 52,6 |
| 30. CI 42090 | 0 | 0 | 0 | 0 | 0 | 0,0002 |
| antioxidant, pHadjustments, parfum | qs | qs | qs | qs | qs | qs. |
| Water | Ad 100 | Ad 100 | 0 | Ad 100 | Ad 100 | Ad 100 |

| | | | | | | |
|---|---|---|---|---|---|---|
| * nicht erfindungsgemäßes Beispiel | | | | | | |

### Handelsnamen der verwendeten Komponenten

- 1.: Dehyton AB 30, Cognis
- 2.: Speziol C16-C18 Pharma, Cognis
- 3.: Citric Acid Monohydrate fine granular 04 32776, DSM Nutritional Products Europe
- 4.: Dehyquart F 75, Cognis
- 5.: Varisoft PATC, Evonic Goldschmidt
- 6.: Tylose E 707002, SE Tylose
- 7.: Nipagin M, Clariant
- 8.: Hydantol 55 KC, KCl
- 9.: Nipasol M, Clariant
- 10.: Natriumbenzoat Granulat EP/USP, Univar
- 11.: Purac PF90, Purac
- 12.: Suprasel fine, Akzo Nobel
- 13.: Salcare SC 95, Ciba
- 14.: Dehyquart A-CA, Cognis
- 15.: Nacol 16-95, Sasol
- 16.: Lanett 18, Cognis
- 17.: Mirasil DM 100, Bluestar Silicones, (+ Bestandteil von DC 593 Fluid)
- 18.: Tego Amid S18, Evonik Goldschmidt
- 19.: 593 Fluid, Dow Corning oder SS 4230 Momentive Performance Materials (Letzteres nur bei example 4; leave-on 1)
593 Fluid hat ein molares M:Q Verhältnis von 1,2 : 1 der Gewichtsanteil an D-Einheiten ist 56 %
SS 4230 hat ein M:Q Verhältnis 0,7 : 1 und es hat eine Molmasse von 31,0 kg/mol.
- 20.: Mirasil CM5, Bluestar Silicones (+ Bestandteil von SS 4230 und 1501 Fluid, Dow Corning)
- 21.: 1501 Fluid, Dow Corning
- 22.: Tegin Iso, Evonik Goldschmidt
- 23.: Tego Alkanol IC 20, Evonik Goldschmidt
- 24.: Refined Glycerin, KAO
- 25.: Tego Betain F 50, Evonik Goldschmidt
- 26.: Ethersulfat K 25 NK, Beiersdorf
- 27.: Ucare Polymer JR 400, Dow Chemical
- 28.: Tegosoft TN, Evonik Goldschmidt
- 29.: Ethanol Azeotrop Ph. Eur. ART 60408, Nedalco
- 30.: FD&C Blue No. 1, BASF

### Herstellanweisungen:

### Shampoo (Beispiel 12)

1. Sodium Benzoate, Cocamidopropyl Betaine, Sodium Laureth Sulfate und Wasser zusammengeben (Tensidphase)
2. Polyquaternium-10 in etwas Wasser einstreuen, quellen lassen und zur Tensidphase geben
3. Trimethylsiloxysilicate und Dimethicone zur Tensidphase geben
4. Parfum zugeben
5. pH-Wert auf ca. 5 einstellen

### Rinse-off Conditioner (Beispiel 1, 2, 3, 8)

1. Fettalkohole, Propylparaben und Stearamidopropyl Dimethylamine im Wasserbad aufschmelzen
2. Methylparaben, Natrium Chloride, Lactic Acid, Coco Betaine und Wasser auf 80 °C erhitzen
3. Phasen vereinigen; bei 60 °C homogenisieren
4. Dimethicone und Trimethylsiloxysilicate bei 40°C zum Ansatz geben
5. Parfüm bei Raumtemperatur zum Ansatz geben

### Leave-an treatment (Beispiel 4, 5)

1. Glyceryl Isostearate, Isoceteth-20, Trimethylsiloxysilicate, Dimethicone bzw. Cyclomethicone in ein Becherglas vorlegen, auf 80 °C hochheizen
2. Parfum kalt zur heissen Fettphase geben
3. Glycerin, DMDM Hydantoin und Wasser auf 80 °C hochheizen, zur Fettphase geben und unter schnellem rühren abkühlen lassen

### Leave-on treatment (Beispiel 6, 7, 10)

1. Trimethylsiloxysilicate, Dimethicone, Dimethiconol und Cyclomethicone zusammen geben und rühren

### Leave-on treatment (Beispiel 9)

1. DMDM Hydantoin, Cetrimonium Chloride und Wasser unter rühren zusammengeben, pH -Wert auf ca. 4,7 einstellen
2. Dimethicone, Trimethylsiloxysilicate mischen, dann Polyquaternium-37 zugeben, glattrühren, zur Wasserphase geben und kalt homogenisieren

### Leave-on treatment (Beispiel 11)

1. Cetearyl Alkohol ,Methylparaben und Propylparaben im Wasserbad aufschmelzen
2. Citronensäure, Glycerin , Distearoylethyl Hydroxyethylmonium Methosulfate, Palmitamidopropyltrimonium Chloride und Wasser auf 80°C ehitzen
3. Beide Phasen zusammengeben und bei 55 °C homogenisieren
4. Hydroxypropyl Methylcellulose bei 50 °C zugeben
5. Dimethicone, Trimethylsiloxysilicate und Cyclomethicone bei 40 °C zugeben
6. Parfum bei Raumtemperatur zugeben

### Leave-on treatment (Beispiel 13)

1. Dimethicone, Cyclomethicone, Trimethylsiloxysilicate und C12-15 Alkyl Benzoate zusammen geben und rühren
2. Parfüm dazu geben
3. Alcohol denat. Citric Acid, Sodium Benzoate, Cl 42090 und Wasser zusammen geben, rühren lassen und zur ersten Phase geben

### Ergebnisse

Die 3 Conditioner-Formulierungen (Beispiel 1, 2 und 3) wurden auf ihre Colorschutzwirkung und ihre Sensorik getestet.

Um die Colorschutzwirkung zu testen, wurden zunächst Haarsträhnen (Imhair Virgin white) mit einer handelsüblichen Haartönung (Alcina Color Emulsion 6.55 dunkelblond-rot-Extra) gefärbt und deren Farbe mittels LAB-Farbmessgerät gemessen. Anschließend wurden die Strähnen jeweils 4 Mal mit Shampoo gewaschen und dann mit der jeweiligen Conditioner-Formulierung behandelt. Nach jedem Wasch/Pflegezyklus wurden die Strähnen mindestens 24 Stunden zum Trocknen aufgehängt, bevor das nächste Mal behandelt wurde.

**ΔE-Werte der rinse-off Conditioner**

| ΔE-Wert | Conditioner 1 (2,5 % TMS) | Conditioner 2 (5 % TMS) | Conditioner 3 |
|---|---|---|---|
| Wert | 13,53 | 12,72 | 14,59 |
| Wert | 13,47 | 12,60 | 14,69 |

Die Ergebnisse zeigen, dass der Rohstoff Trimethylsiloxysilicate (Silikonharz) aus rinse-off Conditionern einen messbaren (und auch sichtbaren) Farbschutz vor dem Auswaschen gewährleistet, d. h. es ist nach Behandlung mit der Silikonharztechnologie noch mehr Farbe im Haar verglichen mit der Anwendung mit einem konventionellen Conditioner. Auch steigert sich der farbschützende Effekt mit Erhöhung des Gehalts an Trimethylsiloxysilicate.

Zusätzlich zur Untersuchung der Farbschutzleistung wurde die sensorische Performance in einem Halbseitenvergleich im Haarstudio an 10 Personen getestet. In einer Scala von 1 - 10 wurden verschiedene Parameter (siehe Tabelle) von ausgebildeten Friseuren bewertet.

Man sieht, dass die sensorische Performance von Conditioner 1 (mit 2,5 % Trimethylsiloxysilicate) und Conditioner 3 (ohne Trimethylsiloxysilicate) vergleichbar ist; in folgenden Parametern wurde sogar ein Vorteil für die Spülung mit Silikonharz gesehen: Verteilbarkeit, Ausspülbarkeit, Entwirrbarkeit, Kämmbarkeit, Gleitvermögen, Haarstruktur Spitzen im nassen Haar und Entwirrbarkeit, Kämmbarkeit, Haarstruktur Längen und Spitzen im trockenen Haar.

| | Conditioner 1 (2,5 % TMS) | Conditioner 3 |
|---|---|---|
| Verteilbarkeit | 7 | 6,8 |
| Gehalt beim Verteilen | 6 | 6,2 |
| Gleitvermögen beim Verteilen | 6,6 | 7,2 |
| glitschig | 2 | 0 |
| Gehalt beim Ausspülen | 4 | 4,4 |
| Gleitvermögen beim Ausspülen | 7,2 | 7,8 |
| Ausspülbarkeit | 6,8 | 5,8 |
| weich/ geschmeidig | 1 | 0 |
| Gleitvermögen nach 40 s ausspülen | 6 | 6,6 |
| Entwirrbarkeit nass | 7 | 6,8 |
| Kämmbarkeit nass | 7,2 | 7 |
| Gleitvermögen nass | 7 | 6,8 |
| Haarstruktur nass Längen | 7 | 7 |
| Haarstruktur nass Spitzen | 6,2 | 6 |
| kein Belag | 4 | 4 |
| Föhn/ Formbarkeit optimal | 3 | 4 |
| Frisurenfülle | 6 | 6,6 |
| Entwirrbarkeit trocken | 7,2 | 6,6 |
| Kämmbarkeit trocken | 7,6 | 7 |
| Gleitfähigkeit trocken | 7,8 | 7,8 |
| Struktur Längen trocken | 7,6 | 7,4 |
| Struktur Spitzen trocken | 6,6 | 6,4 |
| keine Aufladung | 5 | 5 |
| weich/ geschmeidig trocken | 2 | 0 |

Durch Aufnahmen mit dem confokalen Laser Scanning Mikroskop kann beobachtet werden, dass die Spülung mit Trimethylsiloxysilicate (Conditioner 2; 5 % TMS) im Gegensatz zum Conditioner ganz ohne Silikonharz einen wasserresistenten Film auf dem Haar bilden kann, was vermutlich der Grund dafür ist, dass die Haarfarbe nicht so schnell ausblutet.

Auch leave-on Formulierungen mit Trimethylsiloxysilicate wurden auf ihren Farbschutz getestet. Hierbei wurden zwei verschiedene Applikationsformen gewählt; eine leichte Sprayvariante und ein pflegendes Serum auf Silikonbasis.

Die Sprays (zwei verschiedene Varianten, leave-on 1, leave-on 2) wurden gegen ein handelsübliches Pflegespray (Nivea Diamond Gloss Spray) getestet und schneiden in der Farbschutzleistung besser ab als eben dieses.

Zwei Seren (leave-on 3 mit Trimethylsiloxysilicate; leave-on 4 ohne Trimethylsiloxysilicate) wurden ebenfalls auf die Farbschutzleistung getestet und auch in dieser Applikationsform schneidet das Serum mit TMS besser ab. (Anmerkung zur Testdurchführung: im Gegensatz zu Conditioner und Spray wurde das Serum nur nach jeder dritten Wäsche aufgetragen, was dem Verbraucherverhalten am Nächsten kommt.).

**ΔE-Werte der leave-on Sprayformulierungen**

| ΔE-Wert | handelsübliches leave-on Spray | Leave-on 1, 2,5 % TMS in Cyclomethicone | Leave-on 2, 2,5 % TMS in Dimethicone |
|---|---|---|---|
| Wert | 11,66 | 8,82 | 9,88 |
| Wert | 13 | 8,90 | 9,77 |

**ΔE-Werte der leave-on Serumformulierungen**

| ΔE-Wert | Serum (leave-on 4) | Serum mit 2,5 % TMS (Leave-on 3) |
|---|---|---|
| Wert | 11,61 | 7,39 |
| Wert | 12,20 | 7,28 |

### Beispiele Mischung aus Trimethylsiloxysilicate und Polypropylsilsesquioxane

| INCI | w/w % (active content) | | | | | | | |
|---|---|---|---|---|---|---|---|---|
| | 14* (rinse-off Conditio ner 5) | 15*** (leave-on 8) | 16* leave -on 9) | 17* (Sham poo 2) | 18* (Leave -on 10 | 19* (Leav e-on 11) | 20* (Leave -on 12) | 21** (Leave-on 13) |
| 1.Coco Betaine | 0,26 | 0 | 0 | 0 | 0 | 0 | 0 | 0 |
| 2, Cetearyl Alkohol | 0 | 0 | 0 | 0 | 2,8 | 0 | 0 | 0 |
| 3. Citric Acid | 0 | 0 | 0 | 0 | 0,01 | 0 | 0,05 | 0 |
| 4. Distearoylethyl Hydroxyethylmonium Methosulfate + Cetearyl Alkohol | 0 | 0 | 0 | 0 | 2 | 0 | 0 | 0 |
| 5. Palmitamidopropyltrimo nium Chloride + Propylene Glycol | 0 | 0 | 0 | 0 | 1 | 0 | 0 | 0 |
| 6. Hydroxypropyl Methylcellulose | 0 | 0 | 0 | 0 | 0,25 | 0 | 0 | 0 |
| 7. Methylparaben | 0,25 | 0 | 0 | 0 | 0,25 | 0 | 0 | 0 |
| 8. DMDM Hydantoin | 0 | 0 | 0,6 | 0 | 0 | 0,6 | 0 | 0 |
| 9. Propylparaben | 0,1 | 0 | 0 | 0 | 0,1 | 0 | 0 | 0 |
| 10. Sodium Benzoate | 0 | 0 | 0 | 0,4 | 0 | 0 | 0,4 | 0 |
| 11. Lactic Acid | 0,72 | 0 | 0 | 0 | 0 | 0 | 0 | 0 |
| 12. Sodium Chloride | 0,01 | 0 | 0 | 0 | 0 | 0 | 0 | 0 |
| 13. Polyqquaternium-37 | 0 | 0 | 0 | 0 | 0 | 2 | 0 | 0 |
| 14. Cetrimonium Chloride | 0 | 0 | 0 | 0 | 0 | 0,6 | 0 | 0 |
| 15. Cetyl Alkohol | 2,2 | 0 | 0 | 0 | 0 | 0 | 0 | 0 |
| 16. Stearyl Alkohol | 4,4 | 0 | 0 | 0 | 0 | 0 | 0 | 0 |
| 17. Dimethicone | 5,4 | 4,6 | 4,6 | 5,4 | 5 | 4,6 | 4,6 | 0 |
| 18. Stearamidopropyl Dimethylamine | 2,2 | 0 | 0 | 0 | 0 | 0 | 0 | 0 |
| 19. Cyclomethicone | 0 | 87,6 | 0 | 0 | 0,5 | 0 | 25,4 | 92,25 |
| 20. Dimethiconol | 0 | 5,2 | 0 | 0 | 0 | 0 | 0 | 5,2 |
| 21. Glyceryl Isostearate | 0 | 0 | 2,2 | 0 | 0 | 0 | 0 | 0 |
| 22. Isoceteth-20 | 0 | 0 | 5 | 0 | 0 | 0 | 0 | 0 |
| 23. Glycerin | 0 | 0 | 2,6 | 0 | 8,7 | 0 | 0 | 0 |
| 24. Cocamidopropyl Betaine | 0 | 0 | 0 | 3 | 0 | 0 | 0 | 0 |
| 25. Sodium Laureth Sulfate | 0 | 0 | 0 | 9 | 0 | 0 | 0 | 0 |
| 26. Polyquaternium-10 | 0 | 0 | 0 | 0,3 | 0 | 0 | 0 | 0 |
| 27. blend of Trimethylsiloxysilicate and Polypropylsilsesquioxan e | 2,5 | 2,5 | 2,5 | 2,5 | 2,5 | 2,5 | 2,5 | 2,5 |
| 28. C12-15 Alkyl Benzoate | 0 | 0 | 0 | 0 | 0 | 0 | 3,5 | 0 |
| 29. Alcohol Denat. | 0 | 0 | 0 | 0 | 0 | 0 | 52,6 | 0 |
| 30. Cl 42090 | 0 | 0 | 0 | 0 | 0 | 0 | 0,0002 | 0 |
| antioxidant, pHadjustments, parfum | qs | qs | qs | qs | qs. | qs. | qs. | 0 |
| Water | Ad 100 | 0 | Ad 100 | Ad 100 | Ad 100 | Ad 100 | Ad 100 | 0 |

| | | | | | | | | |
|---|---|---|---|---|---|---|---|---|
| * nicht erfindungsgemäßes Beispiel ** Vergleichsbeispiel *** erfindungsgemäßes Beispiel | | | | | | | | |

**Handelsnamen der verwendeten Produkte**
- 1.: Dehyton AB 30, Cognis
- 2.: Speziol C16-C18 Pharma, Cognis
- 3.: Citric Acid Monohydrate fine granular 04 32776, DSM Nutritional Products Europe
- 4.: Dehyquart F 75, Cognis
- 5.: Varisoft PATC, Evonic Goldschmidt
- 6.: Tylose E 707002, SE Tylose
- 7.: Nipagin M, Clariant
- 8.: Hydantol 55 KC, KCl
- 9.: Nipasol M, Clariant
- 10.: Natriumbenzoat Granulat EP/USP, Univar
- 11.: Purac PF90, Purac
- 12.: Suprasel fine, Akzo Nobel
- 13.: Salcare SC 95, Ciba
- 14.: Dehyquart A-CA, Cognis
- 15.: Nacol 16-95, Sasol
- 16.: Lanett 18, Cognis
- 17.: Mirasil DM 100, Bluestar Silicones, (+ Bestandteil von DC 593 Fluid)
- 18.: Tego Amid S18, Evonik Goldschmidt
- 19.: Mirasil CM5, Bluestar Silicones (+ Bestandteil von SS 4230 und 1501 Fluid, Dow Corning)
- 20.: 1501 Fluid, Dow Corning
- 21.: Tegin Iso, Evonik Goldschmidt
- 22.: Tego Alkanol IC 20, Evonik Goldschmidt
- 23.: Refined Glycerin, KAO
- 24.: Tego Betain F 50, Evonik Goldschmidt
- 25.: Ethersulfat K 25 NK, Beiersdorf
- 26.: Ucare Polymer JR 400, Dow Chemical
- 27.: MQ 1640, Dow Corning;
MQ 1640 hat ein M : Q Verhältnis von 0,97: 1 und einen Gewichtsanteil an T-Einheiten von 37 %
- 28.: Tegosoft TN, Evonik Goldschmidt
- 29.: Ethanol Azeotrop Ph. Eur. ART 60408, Nedalco
- 30.: FD&C Blue Nr. 1, BASF

### Herstellanweisungen:

### rinse-off canditfoner (Beispiel 14)

1. Fettalkohole, Propylparaben, Stearamidopropyl Dimethylamine und blend aus Trimethylsiloxysilicate und Polypropylsilsesquioxane im Wasserbad aufschmelzen
2. Methylparaben, Natrium Chloride, Lactic Acid, Coco Betaine und Wasser auf 80 °C erhitzen
3. Phasen vereinigen; bei 60 °C homogenisieren
4. Dimethicone bei 40°C zum Ansatz geben
5. Parfüm bei Raumtemperatur zum Ansatz geben

### Leave-on treatment (Beispiel 15)

1. Blend aus Trimethylsiloxysilicate und Polypropylsilsesquioxane in Dimethicone lösen
2. Dimethiconol und Cyclomethicone dazugeben und rühren lassen

### Leave-on treatment (Beispiel 21)

1. Blend aus Trimethylsiloxysilicate und Polypropylsilsesquioxane in Cyclomethicone lösen
2. Dimethiconol dazugeben und rühren lassen

### Leave-on treatment (Beispiel 16)

1. Blend aus Trimethylsiloxysilicate und Polypropylsilsesquioxane in Dimethicone lösen
2. Glyceryl Isostearate undlsoceteth-20 dazu geben, in ein Becherglas vorlegen, auf 80 °C hochheizen
3. Parfum kalt zur heißen Fettphase geben
4. Glycerin, DMDM Hydantoin und Wasser auf 80 °C hochheizen, zur Fettphase geben und unter schnellem rühren abkühlen lassen

### Shampoo (Beispiel 17)

1. Sodium Benzoate, Cocamidopropyl Betaine, Sodium Laureth Sulfate und Wasser zusammengeben (Tensidphase)
2. Polyquaternium-10 in etwas Wasser einstreuen, quellen lassen und zur Tensidphase geben
3. Blend aus Trimethylsiloxysilicate und Polypropylsilsesquioxane in Dimethicone lösen und zur Tensidphase geben
4. Parfum zugeben
5. pH-Wert auf ca. 5 einstellen

### Leave-on Treatment (Beispiel 18)

1. Cetearyl Alkohol, Methylparaben und Propylparaben im Wasserbad aufschmelzen
2. Citronensäure, Glycerin , Distearoylethyl Hydroxyethylmonium Methosulfate, Palmitamidopropyltrimonium Chloride und Wasser auf 80°C ehitzen
3. Beide Phasen zusammengeben und bei 55 °C homogenisieren
4. Hydroxypropyl Methylcellulose bei 50 °C zugeben
5. Blend aus Trimethylsiloxysilicate und Polypropylsilsesquioxane in Dimethicone lösen und zusammen mit Cyclomethicone bei 40 °C zum Ansatz geben
6. Parfum bei Raumtemperatur zugeben

### Leave-on treatment (Beispiel 19)

1. DMDM Hydantoin, Cetrimonium Chloride und Wasser unter rühren zusammengeben, pH -Wert auf ca. 4,7 einstellen
2. Blend aus Trimethylsiloxysilicate und Polypropylsilsesquioxane in Dimethicone lösen, dann Polyquaternium-37 zugeben, glattrühren, zur Wasserphase geben und kalt homogenisieren

### Leave-on treatment (Beispiel 20)

1. Blend aus Trimethylsiloxysilicate und Polypropylsilsesquioxane in Dimethicone lösen, dann mit Cyclomethicone und C12-15 Alkyl Benzoate zusammen geben und rühren
2. Parfüm dazu geben
3. Alcohol denat. Citric Acid, Sodium Benzoate, Cl 42090 und Wasser zusammen geben, rühren lassen und zur ersten Phase geben

### Ergebnisse

Neben dem Trimethylsiloxysilicate allein wurde eine Silikonharzmischung (63 % Trimethylsiloxysilicate; 37 % Polypropylsilsesquioxane[s1]) in leave-on Seren und rinse-off Conditionern getestet.

Die Methodik zur Ergebnisevaluierung entspricht der schon beschriebenen Methodik unter *Beispiele und Ergebnisse (Trimethylsiloxysilicate allein).*

Man sieht, dass das Serum mit dem blend aus Trimethylsiloxysilicate und Polypropylsilsesquioxane (leave-on 8) eine viel bessere Farbschutzleistung gewährleistet als das Serum ohne Silikonharze (leave-on 4).

**ΔE-Werte der leave-on-Serum-Formulierungen mit MQ/TPr-Silikonharzen**

| ΔE-Wert | Serum (leave-on 4) | Serum mit 2,5 % TMS /PSO (leave-on 8) |
|---|---|---|
| Wert | 20,32 | 8,88 |
| Wert | 20,21 | 9,30 |

Desweiteren ist aufgefallen, dass der Rohstoff Dimethicone (welcher in den Seren standardmäßig eingesetzt wurde) auch einen positiven Einfluss auf das Farbschutzergebnis hat. Lässt man diesen weg, so verschlechtert sich das Farbschutzergebnis.

**ΔE-Werte der leave-on-Serum-Formulierungen mit MQ-TPr-Silikonharzen mit und ohne Dimethicone**

| ΔE-Wert | Serum mit 2,5 % TMS/PSO und 4,6% Dimethicone (leave-on 8) | Serum mit 2,5 % TMS/PSO ohne Dimethicone (leave-on 13) |
|---|---|---|
| Wert | 8,88 | 13.20 |
| Wert | 9,30 | 11,58 |

Außerdem wurde getestet, ob das Serum mit besagten Silikonharzen auch einen Farbschutzeffekt auf oxidativ gefärbten Haaren leisten kann. Hierzu wurde eine rot färbende, oxidative Haarfarbe (P-Amino-O-Cresol, P-Aminophenol) benutzt.

Das Ergebnis zeigt, dass ein Farbschutzeffekt auch hier möglich ist.

**ΔE-Werte der leave-on Serum-Formulierungen mit MQ/TPr-Silikonharzen (Performance auf oxidativ gefärbten Haaren)**

| ΔE-Wert | Ohne Serum | Serum (leave-on 4) | Serum mit 2,5 % TMS/PSO (leave-on 8) |
|---|---|---|---|
| Wert | 3,87 | 3,49 | 1,97 |
| Wert | 3,62 | 4,05 | 1,05 |

Desweiteren wurden auch Conditioner mit besagter Silikonharzmischung auf ihren Farbschutzeffekt hin geprüft.

Betrachtet man die ΔE-Werte, so sieht man, dass der Conditioner mit der Mischung aus Trimethylsiloxysilicate und Polypropylsilsesquioxane (Conditioner 5) einen besseren Farbschutzeffekt gewährleistet als der handelsübliche Conditioner für coloriertes Haar.

**ΔE-Werte von rinse-off Conditionern mit MQ/TPr-Silikonharzen**

| ΔE-Wert | Handelsüblicher Conditioner für coloriertes Haar | Conditioner 5 (mit 2,5 % TMS/PSO) |
|---|---|---|
| Wert | 17,38 | 14,55 |
| Wert | 18,65 | 15,23 |

Es wurde hier ebenfalls ein sensorischer Vergleich an zehn Köpfen im Haarstudio durchgeführt.

Für den Conditioner mit der Mischung aus Trimethylsiloxysilicate und Polypropylsilsesquioxane ergeben sich im Vergleich zu Conditioner 3 ohne Silikonharze Vorteile in folgenden Parametern:
Verteilbarkeit, Gleitvermögen beim Verteilen, Weichheit/ Geschmeidigkeit beim Ausspülen, Entwirrbarkeit, Kämmbarkeit, Gleitvermögen, Haarstruktur in Längen und Spitzen im nassen Haar und Glanz und Haarstruktur in den Längen im trockenen Haar.

### Haarstudioergebnis Conditionervergleich (mit und ohne MQ/TPr-Silikonharz)

| | conditioner 5 (mit 2,5 % TMS/PSO) | Conditioner 3 |
|---|---|---|
| Verteilbarkeit | 7 | 6,7 |
| Gehalt beim Verteilen | 4,2 | 5,1 |
| glitschig | 2 | 0 |
| Gleitvermögen, beim Verteilen | 6 | 5,9 |
| Gehalt beim Ausspülen | 3,3 | 3,8 |
| Gleitvermögen beim Ausspülen | 6,3 | 6,7 |
| glitschig beim Ausspülen | 3 | 2 |
| Ausspülbarkeit | 7 | 7 |
| weich/ geschmeidig beim Ausspülen | 1 | 0 |
| Gleitvermögen nach 40 s Ausspülen | 5,7 | 5,7 |
| Entwirrbarkeit nass | 6,1 | 5,5 |
| Kämmbarkeit nass | 6,8 | 6,4 |
| Gleitvermögen nass | 6,8 | 6,1 |
| Haarstruktur nass Längen | 6,8 | 6,3 |
| Haarstruktur nass Spitzen | 5,8 | 5,1 |
| kein Belag | 10 | 10 |
| weich/ geschmeidig nass | 2 | 0 |
| Föhn/ Formbarkeit | 10 | 10 |
| Frisurenfülle | 6,1 | 6,2 |
| Glanz | 6,4 | 6,3 |
| Entwirrbarkeit trocken | 6,3 | 6,4 |
| Kämmbarkeit trocken | 6,8 | 7 |
| Gleitfähigkeit trocken | 6,9 | 7,1 |
| Struktur Längen trocken | 7,2 | 7 |
| Struktur Spitzen trocken | 6,2 | 6,2 |
| keine Aufladung | 9 | 9 |
| weich/geschmeidig trocken | 1 | 2 |

## Patentansprüche

1. Verwendung von Haarbehandlungsmitteln enthaltend
i) 0 bis 1 Gew.-% anionische Tenside,
ii) 0,5 bis 5 Gew.-% Silikonharze
wobei die Silikonharze
a) M- und Q-Monomereinheiten im molaren Verhältnis M:Q = 0,4 : 1 bis 1,2 : 1 enthalten,
b) eine Molmasse von 12 bis 35 kg/mol, besonders bevorzugt 15 bis 32 kg/mol haben
c) und zusätzlich T- oder D-Monomereinheiten enthalten,
wobei die Silikonharze in Form von Trimethylsiloxysilicaten und Polypropylsilsesquioxane vorliegen,
wobei die Gesamtmenge an Silikonharz im Bereich von 0,5 bis 5 Gew.-% liegt,
iii) Polydimethylsiloxane,
iv) 0 bis 0,5 Gew.-% Wasser,
zum Schutz der Farbe künstlich gefärbter Haare vor dem Verblassen der Farbe.

2. Verwendung von Haarbehandlungsmitteln nach Anspruch 1 **dadurch gekennzeichnet, dass** das Silikonharz M- und Q- und entweder T- oder D-Monomereinheiten enthält, deren molares Verhältnis sich wie M:Q 0,5 : 1 bis 1 : 1 verhält und der Gewichtsanteil an D- oder T-Einheiten 30 bis 50 Gew.-% beträgt.

## Claims

1. Use of hair treatment compositions comprising
i) 0 to 1 wt% anionic surfactants
ii) 0.5 to 5 wt% silicone resins
wherein the silicone resins
a) comprise M and Q monomer units in a molar ratio of M:Q = 0.4:1 to 1.2:1,
b) have a molar mass of 12 to 35 kg/mol, particularly preferably 15 to 32 kg/mol
c) and additionally comprise T or D monomer units
wherein the silicone resins are in the form of trimethylsiloxysilicates and polypropylsilsesquioxanes,
wherein the total amount of silicone resin is in the range of 0.5 to 5 wt%.
iii) polydimethylsiloxanes,
iv) 0 to 0.5 wt% water,
for protecting the colour of the artificially dyed hair against fading of the dye.

2. Use of hair treatment compositions according to Claim 1, **characterized in that** the silicone resin comprises M and Q and either T or D monomer units, of which the molar ratio M:Q is from 0.5:1 to 1:1 and the proportion by weight of D or T units is 30 to 50 wt%.

## Revendications

1. Utilisation d'agents de traitement des cheveux, contenant :
i) 0 à 1 % en poids de tensioactifs anioniques,
ii) 0,5 à 5 % en poids de résines de silicone,
les résines de silicone
a) contenant des unités monomères M et Q en un rapport molaire M:Q = 0,4:1 à 1,2:1,
b) ayant une masse molaire de 12 à 35 kg/mol, de manière particulièrement préférée de 15 à 32 kg/mol,
c) et contenant en outre des unités monomères T ou D,
les résines de silicone se présentant sous la forme de triméthylsiloxysilicates et de polypropylsilsesquioxane, la quantité totale de résine de silicone se situant dans la plage allant de 0,5 à 5 % en poids,
iii) des polydiméthylsiloxanes,
iv) 0 à 0,5 % en poids d'eau,
pour la protection de la couleur de cheveux colorés artificiellement contre la décoloration de la couleur.

2. Utilisation d'agents de traitement des cheveux selon la revendication 1, **caractérisée en ce que** la résine de silicone contient des unités monomères M et Q et T ou D, dont le rapport molaire M:Q est de 0,5:1 à 1:1, la proportion en poids des unités D ou T étant de 30 à 50 % en poids.
